# EUROPEAN PATENT SPECIFICATION

(11) **EP 4 076 330 B1**
(45) Date of publication and mention of the grant of the patent: **17.01.2024**
(21) Application number: 20830339.6
(22) Date of filing: 21.12.2020
(51) Int. Cl.: A61H 1/02

(54) **A BALANCE DEVICE WITH A PIVOTABLE PLATE**
AUSGLEICHSVORRICHTUNG MIT EINER SCHWENKBAREN PLATTE
DISPOSITIF D'ÉQUILIBRE DOTÉ D'UNE PLAQUE PIVOTANTE

(30) Priority: 20.12.2019 IT 201900025075
(43) Date of publication of application: 26.10.2022
(73) Proprietor: Delos S.r.l., 10145 Torino (IT); Riva Violetta, Dario, 10146 Torino (IT)
(72) Inventor: RIVA VIOLETTA, Dario, 10146 Torino (IT)
(74) Representative: Savoca, Agatino
(86) International application number: PCT/IB2020/062278
(87) International publication number: WO 2021/124308

(56) References cited:
- EP-A1- 0 691 837
- US-A- 5 372 563
- US-A1- 2003 036 462
- US-A1- 2003 236 151

## Description

### Technical field

This invention is generally in the field of postural rehabilitation devices; in particular, the invention relates to a balance plate device.

### Prior art

Postural instability, falls and loss of motor independence of the elderly population are a growing problem in developed countries. At the same time, in modern sport there is a progressive increase in injuries. Among the most common injuries are ankle and knee sprains, lower back pain, plantar fasciitis and Achilles tendonitis.

The fact that 80% of human movements occur in single stance helps to explain how single stance instability is an important factor in the genesis of lower limb injuries, back pain and falls and becomes a key element in prevention. Instability depends on the lack of proprioceptive control that inevitably develops in every individual, young or old, athlete or sedentary, when the interaction of the foot with uneven ground is reduced. An inadequate proprioceptive control makes every motor action, which arises from the contact of the foot with the ground less effective, less safe, and more micro-traumatic on joint and musculo-tendinous structures, both in common daily activities and in the movements of athletes. A limited mobility of the tibiotarsal and subtalar joints is one of the first consequences of interaction with predominantly flat ground and is one of the most important factors in the causal chain leading to the regression of proprioceptive control.

Devices for foot rehabilitation and balance training are known, in the form of balance plates or "proprioceptive tablets," which use a tilting plate (with at least two degrees of freedom), or oscillating plate with a single degree of freedom, resting on the floor.

An example of this solution is known from the document EP0862930 A1, which describes a plate oscillating about a movable axis of rotation, this plate being provided with an upper plane for supporting one or both feet of a user and a pair of underlying rolling arches in contact with a fixed support plane, shaped as a circular cylinder section in such a way that, during the tilting movement of the plate, the axis of rotation of said plate is movable in space and positioned above the support plane of the foot/feet.

The mechanical features allow the subject to control the movements of the support plane of the foot in all the degrees of inclination. The controllability depends on the fact that the rotation axis and the contact point of the rolling elements are aligned on the same vertical line, both with each other and with the virtual center of rotation of the ankle. In such a device, the instability is of an intrinsic type (i.e., induced by the user's movements) and allows the subject to pass from macro-instability with high postural disorder to micro-instability and refined control, both postural and of the tilting support base of the foot/feet. The possibility of prioritizing specific segments of the joint range or of excluding other less favorable ones could favor the persistence of areas with reduced proprioceptive control.

A different example of a postural rehabilitation device is known from the document WO 2014/085732 A1, wherein is provided a motor adapted to assist the oscillation of the platform by engaging toothed profiles, movable integrally with the platform, with a pinion operated by the motor. In this way, an extrinsic instability (i.e., induced by a motor) is created, which allows the user to intervene on the platform, regulating the oscillation thereof. A similar solution is proposed in the document US 5 372 563 A.

However, the configuration of the toothed profiles (which face the floor, and not the support plane of the user's feet) is such that the large space below the platform, required for housing the gearmotor, involves an excessive height of said platform with respect to the ground level. In effect, the toothing of the toothed profiles is of the external type, i.e. it does not face the platform, which involves the need to place the gearmotor under the platform, increasing the vertical dimensions of the device and the consequent excessive elevation of the platform with respect to the ground.

The elevation of the level of the support plane of the feet and the consequent raising of the plane of the eyes compromise the stabilizing action of the visual system by changing the relationship with the surrounding environment. Thus, arises the need to reduce the height of the platform from the ground as much as possible to allow visual coupling to anchor points present in the environment and allow the view to play the role of secondary stabilizer.

A further example of a postural rehabilitation device, adapted to induce an extrinsic instability, is known from the document US 2003/236151 A1, wherein the platform on which the user's feet rest is made to oscillate with respect to a fixed support by means of a U-shaped profile hinged to a first toothed wheel that engages a second toothed wheel, which rotates an eccentric pin, which is slidable within a groove provided in the U-shaped profile. However, due to the peculiar configuration of this transmission, formed by the toothed wheels interposed between the fixed support and the U-shaped profile, the instantaneous axis of rotation of the plate is positioned below the support plane of the user's feet.

The presence of a center of rotation placed below the support plane of the foot or feet involves, when this surface is not horizontal, a horizontal and vertical displacement of the virtual center of rotation of the ankle and its consequent misalignment with respect to the vertical line that contains the center of rotation of the instrument.

It is however essential to minimize the horizontal and vertical displacement of the virtual center of rotation of the ankle. The foot-ankle system is the interface with the ground and has as its primary function that of adapting to the different support surfaces, ensuring maximum stability of the upper body masses. The more the stability depends on the proprioceptive reflexes of the stabilizing muscles close to the point of support, the more ergonomic the movements will be and the less there will be a need to introduce compensatory movements of the upper body masses to manage the vertical. To effectively simulate the various types of intervention of the foot-ankle system, it is therefore necessary to administer inclination variations of the support plane of the foot that minimize the horizontal and vertical displacement of the virtual center of rotation of the ankle. In this way it will be possible to propose situations and variations of situations similar to those that may occur in the natural environment on uneven terrain.

In any case, the solutions proposed in the prior art do not allow for such a result to be achieved.

In effect, the solutions of the prior art described above are characterized in that they only allow a double stance support.

The absence of single stance support limits the reflex activation of the stabilizing muscles and prevents adequate tensile stress of the joint capsules and ligaments necessary to allow an increase in their elasticity and resilience, compromising the protective action against spraining events of the ankle and knee and spine stability.

The movement may only take place in the latero-lateral direction, and therefore the antero-posterior movements of plantar and dorsal flexion, necessary for the recovery of joint mobility in the sagittal plane, are not possible.

Furthermore, the double stance support does not allow the exploration of the entire articular range of pronation-supination, limiting, in this case, the recovery of mobility in the frontal plane.

The above limitations, understandably, compromise or significantly affect the effectiveness of the rehabilitation and preventive treatment.

### Summary of invention

An object of this invention is to overcome the aforementioned problems by the technical features of claim 1.

From an evaluation point of view, with a system according to this invention it is desired to measure the effectiveness of the proprioceptive reflexes (proprioceptive control) in the management of vertical stability at the different inclinations of the single stance, double stance or seated support plane, and quantify the intervention of the visual system as a stabilizer. A second evaluation objective is to evaluate the range of motion of the ankle and its influence on stability in single stance. A third evaluation objective is to measure the effectiveness of the combined intervention of the proprioceptive and visual sensory systems in managing the vertical in dynamic situations.

From a rehabilitation and training point of view, one object is to train the effectiveness and endurance of proprioceptive reflexes and induce structural adaptations at the level of ligaments, tendons, joint capsules and bones, by means of stresses that simulate those originating from the interaction of the foot with uneven ground, but may be administered by categories in tens of thousands of repetitions.

Applications of medium- and low-intensity voltages have in effect been shown, in the experimental studies available in the literature, to be able to increase resilience and tolerance to impulsive mechanical stresses (potentially damaging even as a single event) or repetitive (individually non-damaging, but which may become harmful in the case of a high number of repetitions) by the connective tissues.

To obtain these results, a system is provided for the evaluation and training of the joint mobility of the ankle, of the balance capacity and imbalance management skills and for the re-education of proprioceptive reflexes, comprising a destabilizing means capable of generating situations of instability of extrinsic origin (motorized) of the plane on which the individual is in single stance, double stance or seated, and static situations characterized by different inclinations of the support plane, wherein a plate (constituting the support plane of the user) is supported oscillatably by one or more contact members with a fixed support, these members being integrally movable with a toothed sector having the teeth facing a lower surface of the plate. A gear member, driven in rotation by a gearmotor, engages the toothed sector to impart an oscillation to the plate, so that the rotation of the gear member may be controlled in speed and/or position.

This internal arrangement of the toothing of the toothed profile offers two advantages. The first consists in minimizing the height from the ground of the plate, for example by containing it within 11 cm, so as not to compromise the stabilizing action of the visual system, while keeping the relationship with the surrounding environment unaltered.

The second advantage is that of minimizing the horizontal and vertical displacements of the virtual center of rotation of the ankle, in the case of the single stance support, thus keeping it as close as possible to the center of rotation of the instrument.

In effect, the foot-ankle system is the interface with the ground in the earth's gravitational environment, and its primary function is to adapt to the different support surfaces, ensuring maximum stability of the upper body masses. The more stability depends on the proprioceptive reflexes of the stabilizing muscles near the point of support, the more ergonomic the movements will be and the less there will be a need to introduce compensatory movements of the upper masses.

In order to effectively simulate the various types of intervention of the foot-ankle system, it is therefore necessary to administer variations in the inclination of the support plane of the foot, in the case of the single stance support, which minimize the horizontal and vertical displacement of the virtual center of rotation of the ankle. In this way, it will be possible to propose situations and variations of situations similar to those that may occur in the natural environment on uneven terrain.

In order to minimize the displacement of the center of rotation of the ankle - and preferably to contain the height of the support plane from the ground within a height of 110 mm - the instantaneous axis of rotation of the instrument is fixed in space and placed above the support plane of the foot at a distance expediently between 20 and 80 mm, preferably 50 mm. To satisfy this requirement, a cradle movement may be used.

According to an embodiment, the device may generate inclinations of the support plane (expediently configurable electronically), in the range of ± 20°.

A further, non-negligible advantage is represented by the small size and limited weight that make the device easy to handle and allow the physiotherapist, doctor, osteopath and chiropractor to maintain, if necessary, physical contact with the patient and to use the force of gravity as a joint mobilizer and remodeler, at the same time encouraging the reactivation of the anti-gravity mechanisms of postural stability. This introduces the concept of instrumentally assisted manual therapy for the restoration and development of antigravity efficiency.

The aforesaid and other objects and advantages are achieved, according to an aspect of the invention, by a device having the features defined in claim 1. Preferred embodiments of the invention are defined in the dependent claims.

### Brief description of the drawings

The functional and structural features of some preferred embodiments of a device according to the invention will now be described. Reference is made to the accompanying drawings, wherein:
- Fig. 1 is a schematic perspective view of a balance plate device according to an embodiment of this invention;
- Fig. 2 is a schematic perspective view of the device of Fig. 1, wherein a part of the fixed support is removed, to show one of the contact members which oscillatably support the plate with respect to the fixed support; and
- Fig. 3A and 3B are a longitudinal-sectional and a cross-sectional view respectively of a balance plate device according to an embodiment of the invention, wherein a detail of the contact member, equipped with a toothed profile meshed by a pinion in turn driven by a gear motor, is visible.

### Detailed description

Before describing in detail a plurality of embodiments of the invention, it should be clarified that the invention is not limited in its application to the construction details and configuration of the components presented in the following description or illustrated in the drawings. The invention is capable of assuming other embodiments and of being implemented or constructed in practice in different ways. It should also be understood that the phraseology and terminology have a descriptive purpose and should not be construed as limiting.

Referring by way of example to the figures, a device 9 for evaluating and training the articular mobility of the ankle, the ability to balance and manage imbalance and for re-educating the proprioceptive reflexes, comprises a plate 4, pivotable about at least one axis of rotation and provided with an upper surface 6 for the support of the foot/feet of a user or while seated.

The device 9 further comprises a fixed support 16, adapted to support the plate 4 oscillatably with respect to a support plane P (expediently, the floor of a room wherein the exercises are carried out).

The plate 4 has at least one contact member 10 (expediently, with a circular sector), adapted to support the plate 4 oscillatingly with respect to the fixed support 16, this contact member 10 having a contact profile 10a with said fixed support 16 (and/or with elements rotatably supported by the fixed support 16) such that the instantaneous axis of rotation of the plate 4 is fixed in space and positioned above the upper support plane of the foot/feet 6.

A toothed sector 18 is provided, movable integrally with the contact member 10 and having all the teeth facing the plate 4. For the sake of greater clarity, the toothed sector 18 is configured in such a way that, ideally extending its teeth in length, said teeth would all be centered either in the center of the circular profile of the toothed sector 18 or in that of the contact member 10.

A gear member 20 is also provided, which may rotate around an axis of rotation x, which gear member 20 engages the toothed sector 18 in such a way that a rotation of said gear member 20 causes an oscillation of the toothed sector 18 (and, consequently, of the plate 4), and a gearmotor 22, connected to the gear member 20 and adapted to rotate said gear member 20, so that the rotation of the gear member 20 (and consequently also of the plate 4) is controllable in speed and/or position.

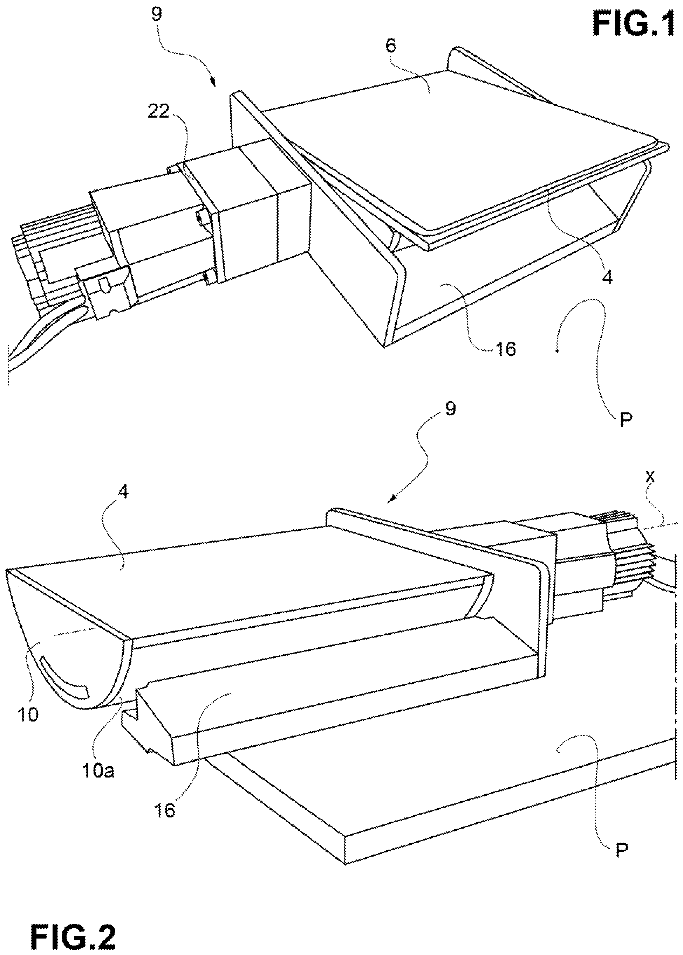

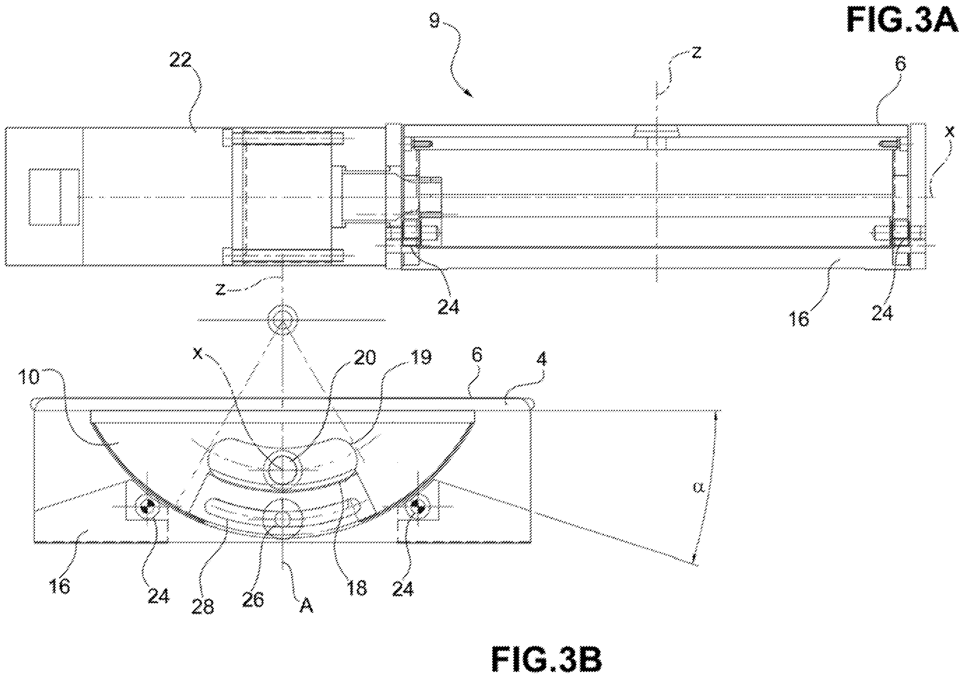

According to a preferred embodiment, the contact member 10 has a cylindrical contact profile 10a with the fixed support 16 (as illustrated by way of example in Fig. 2 and 3B). Such a configuration allows for a cradle-like oscillation to be imparted to the plate 4.

Preferably, this cylindrical contact profile 10a is at least partially circular cylindrical.

According to a preferred embodiment, the contact member 10 is slidably supported on rollers 24, in turn rotatably supported by the fixed support 16 about respective axes of rotation, fixed with respect to said fixed support 16.

Expediently, the rollers 24 are arranged symmetrically with respect to a longitudinal center plane A of the device 9. The longitudinal center plane A of the device 9 expediently contains the instantaneous axis of rotation of the plate 4.

The contact member 10 rests in at least one point on the fixed support 16 and/or in at least two points on the rollers 24.

Expediently, there is a pair of contact members 10, shaped like arches or arched pads.

According to a preferred embodiment, the fixed support 16 comprises a pair of side members, which, facing each other, define a profile at least partially complementary to the contact member 10 (either this is configured as a single cylinder portion, or arched pads supporting the plate 4 are provided), the contact member 10 is supported slidably on these side members. Expediently, these side members may be configured as vertical uprights shaped so as to form, on the respective faces facing the contact member 10, respective seats in a circumferential arc, wherein the contact member 10 supporting the plate 4 is slidably accommodated.

According to an embodiment, the toothed sector 18 is a sector of a circular ring gear.

Preferably, the toothed sector 18 is bound to the contact member 10.

According to an embodiment, illustrated by way of example in Fig. 3B, the toothed sector 18 extends inside an arc-shaped slot 19, made in the contact member 10, into which the slot 19 is inserted the gear member 20, in such a way as to engage the toothed sector 18.

According to an alternative embodiment (not shown), the toothed sector 18 is embossed on a side wall of the contact member 10.

In the illustrated example, the gear member 20 is a pinion.

According to a further embodiment (not shown), the toothed sector 18 (in the form, for example, of the ring gear sector), is positioned on a plane parallel to the plane of the contact member 10, with its center coinciding with the center of said contact member 10.

Expediently, instead of the pinion, a worm gear is provided, moved by the gearmotor 22, this worm gear meshing with the toothed sector 18.

Preferably, a pair of contact members 10 with a cylindrical surface, spaced along the axis of rotation x is provided.

According to an embodiment, the downward decentering of the plate 4, understood as the distance of the support plane of the user (i.e., the upper surface 6) from the instantaneous center of rotation, is between 20 and 80 mm.

Preferably, the distance between the plate 4 and the support plane P is less than 110 mm.

According to an embodiment, the plate 4 is pivotable, with respect to a rest position (that is, a position wherein the plate 4 is in a horizontal configuration), by an angle α between -20° and +20°.

In order to be able to move and lift the entire device, suitable slots 28, concentric to the axis of rotation, may be provided on the two contact members 10, within which cylindrical pins 26, integral with the fixed walls 16, engage.

Various aspects and embodiments of a device according to the invention have been described. It is understood that each embodiment may be combined with any other embodiment. Furthermore, the invention is not limited to the described embodiments, but may be varied within the scope defined by the appended claims.

## Claims

1. Device (9) for the assessment and training of ankle joint mobility, balance capacity and imbalance management skills and re-education of proprioceptive reflexes, comprising:
- a plate (4), oscillatable about at least one axis of rotation and provided with an upper plane (6) for supporting the foot/feet of a user; and
- a fixed support (16), adapted to oscillatably support the plate (4) with respect to a support surface (P);
said plate (4) having at least one contact member (10), adapted to oscillatably support the plate with respect to the fixed support (16), said contact member (10) having a contact profile (10a) in contact with said fixed support (16) in such a way that the instantaneous axis of rotation of the plate (4) is fixed in space and positioned above said upper support plane (6);
**characterized in that** it comprises:
- a toothed sector (18), movable integrally with said contact member (10) and having the teeth turned toward the plate (4);
- a gear member (20), rotatable about a rotation axis (x), said gear member (20) engaging the toothed sector (18) so that a rotation of said gear member (20) causes an oscillation of the toothed sector (18) and of the plate (4); and
- a gearmotor (22), connected to the gear member (20) and adapted to rotate said gear member (20), so that the rotation of the gear member (20) is controllable in speed and/or position.

2. Device according to claim 1, wherein said contact member (10) has a contact profile (10a), at least partially cylindrical, in contact with the fixed support (16).

3. Device according to claim 2, wherein said cylindrical contact profile (10a) is at least partially circular cylindrical.

4. Device according to any of the preceding claims, wherein said contact member (10) is slidably supported on rollers (24), in turn rotatably supported by the fixed support (16) about respective axes of rotation, fixed with respect to said fixed support (16).

5. Device according to claim 4, wherein
the rollers (24) are disposed symmetrically with respect to a longitudinal center plane (A) of the device (9).

6. Device according to any of the preceding claims, wherein a pair of contact members (10) are provided, shaped like arches resting in at least one point on the fixed support (16) and/or in at least two points on the rollers (24).

7. Device according to any of the preceding claims, wherein the fixed support (16) comprises a pair of side members having a profile at least partially complementary to the contact members (10), said contact members (10) being slidingly supported on these side members.

8. Device according to any of the preceding claims, wherein the toothed sector (18) is a sector of a circular ring gear.

9. Device according to any of the preceding claims, wherein said toothed sector (18) is carried by the contact member (10).

10. Device according to claim 9, wherein said toothed sector (18) extends inside an arc-shaped slot (19), arranged in the contact member (10), the gear member (20) inserting into said slot (19) so as to mesh with the toothed sector (18).

11. Device according to one of claims 1 to 9, wherein said toothed sector (18) is embossed on a side wall of the contact member (10).

12. Device according to any of the preceding claims, wherein the gear member (20) is a pinion.

13. Device according to any of the preceding claims, comprising a pair of contact members (10) with cylindrical surface, spaced along the axis of rotation (x).

14. Device according to any of the preceding claims, wherein the lowering of the plate (4), measured by the distance of the support plane from the instantaneous center of rotation, is between 20 and 80 mm, preferably 50 mm.

15. Device according to any of the preceding claims, wherein the distance between the plate (4) and the support surface (P) is less than 110 mm.

16. Device according to any of the preceding claims, wherein the plate (4) is oscillatable, with respect to a rest position, by a measure comprised between ± 20° and ± 25°.

## Patentansprüche

1. Vorrichtung (9) zum Beurteilen und Trainieren der Beweglichkeit des Knöchelgelenks, des Gleichgewichtsvermögens und der Fähigkeiten zur Bewältigung von Gleichgewichtsstörungen und des Wiedererlernens propriozeptiver Reflexe, aufweisend:
- eine Platte (4), die um mindestens eine Rotationsachse verschwenkbar und mit einer oberen Fläche (6) zum Abstützen des Fußes / der Füße eines Benutzers versehen ist; und
- eine feste Auflage (16), die dazu ausgelegt ist, die Platte (4) bezüglich einer Stützoberfläche (P) verschwenkbar abzustützen;
wobei die Platte (4) mindestens ein Kontaktelement (10) aufweist, das dazu ausgelegt ist, die Platte bezüglich der festen Auflage (16) verschwenkbar abzustützen, wobei das Kontaktelement (10) ein Kontaktprofil (10a) aufweist, das derart mit der festen Auflage (16) in Kontakt steht, dass die aktuelle Rotationsachse der Platte (4) räumlich fixiert und oberhalb der oberen Stützfläche (6) angeordnet ist;
**dadurch gekennzeichnet, dass** sie aufweist:
- einen gezahnten Bereich (18), der integral mit dem Kontaktelement (10) beweglich ist und dessen Zähne der Platte (4) zugewandt sind;
- ein um eine Rotationsachse (x) drehbares Zahnradelement (20), wobei das Zahnradelement (20) in den gezahnten Bereich (18) eingreift, so dass eine Drehung des Zahnradelements (20) ein Verschwenken des gezahnten Bereichs (18) und der Platte (4) bewirkt; und
- einen Zahnradmotor (22), der mit dem Zahnradelement (20) verbunden und zum Drehen des Zahnradelements (20) ausgelegt ist, so dass die Drehung des Zahnradelements (20) in Bezug auf dessen Geschwindigkeit und/oder dessen Position steuerbar ist.

2. Vorrichtung nach Anspruch 1, wobei das Kontaktelement (10) ein zumindest teilweise zylindrisches Kontaktprofil (10a) aufweist, das mit der festen Auflage (16) in Kontakt ist.

3. Vorrichtung nach Anspruch 2, wobei das zylindrische Kontaktprofil (10a) zumindest teilweise kreiszylindrisch ist.

4. Vorrichtung nach einem der vorangehenden Ansprüche, wobei das Kontaktelement (10) gleitend auf Rollen (24) gelagert ist, die ihrerseits durch die feste Auflage (16) um entsprechende Rotationsachsen drehbar gelagert und in Bezug auf den festen Träger befestigt sind.

5. Vorrichtung nach Anspruch 4, wobei die Rollen (24) bezüglich einer in Längsrichtung verlaufenden Mittelebene (A) der Vorrichtung (9) symmetrisch angeordnet sind.

6. Vorrichtung nach einem der vorangehenden Ansprüche, wobei ein Paar bogenförmige Kontaktelemente (10) vorgesehen sind, die an mindestens einem Punkt auf der festen Auflage (16) und/oder an mindestens zwei Punkten auf den Rollen (24) aufliegen.

7. Vorrichtung nach einem der vorangehenden Ansprüche, wobei die feste Auflage (16) ein Paar Seitenelemente mit einem Profil, das zumindest teilweise zu den Kontaktelementen (10) komplementär ist, aufweist, wobei die Kontaktelemente (10) verschieblich auf den Seitenelementen gelagert sind.

8. Vorrichtung nach einem der vorangehenden Ansprüche, wobei der gezahnte Bereich (18) ein Bereich eines kreisförmigen Zahnkranzes ist.

9. Vorrichtung nach einem der vorangehenden Ansprüche, wobei der gezahnte Bereich (18) von dem Kontaktelement (10) getragen wird.

10. Vorrichtung nach Anspruch 9, wobei der gezahnte Bereich (18) sich innerhalb eines im Kontaktelement (10) angeordneten bogenförmigen Schlitzes (19) erstreckt, wobei das Zahnradelement (20) in den Schlitz (19) eingesetzt wird, so dass es in den gezahnten Bereich (18) eingreift.

11. Vorrichtung nach einem der Ansprüche 1 bis 9, wobei der gezahnte Bereich (18) an einer Seitenwand des Kontaktelements (10) angebracht ist.

12. Vorrichtung nach einem der vorangehenden Ansprüche, wobei das Zahnradelement (20) ein Ritzel ist.

13. Vorrichtung nach einem der vorangehenden Ansprüche, aufweisend ein Paar von Kontaktelementen (10) mit zylindrischer Oberfläche, die entlang der Rotationsachse (x) beabstandet sind.

14. Vorrichtung nach einem der vorangehenden Ansprüche, wobei die durch den Abstand der Stützfläche von dem aktuellen Drehpunkt gemessene Absenkung der Platte (4) zwischen 20 und 80 mm, bevorzugt 50 mm beträgt.

15. Vorrichtung nach einem der vorangehenden Ansprüche, wobei der Abstand zwischen der Platte (4) und der Stützoberfläche (P) geringer als 110 mm ist.

16. Vorrichtung nach einem der vorangehenden Ansprüche, wobei die Platte (4) bezüglich einer Ruheposition um ein Maß zwischen ± 20° und ± 25° verschwenkbar ist.

## Revendications

1. Dispositif (9) pour l'évaluation et l'entraînement de la mobilité d'articulation d'une cheville, de compétences de capacité d'équilibre et de gestion de déséquilibre et de la rééducation de réflexes proprioceptifs, comprenant :
- une plaque (4), oscillante autour d'au moins un axe de rotation et dotée d'un plan supérieur (6) destiné à supporter le piedlles pieds d'un utilisateur ; et
- un support fixe (16), conçu pour supporter la plaque (4) de manière oscillante par rapport à une surface de support (P) ;
ladite plaque (4) ayant au moins un élément de contact (10), conçu pour supporter de manière oscillante la plaque par rapport au support fixe (16), ledit élément de contact (10) ayant un profil de contact (10a) en contact avec ledit support fixe (16) de sorte que l'axe de rotation instantané de la plaque (4) soit fixé dans l'espace et positionné au-dessus dudit plan de support supérieur (6) ; **caractérisé en ce qu'**il comprend :
- un secteur denté (18), mobile d'un seul tenant avec ledit élément de contact (10) et ayant les dents tournées en direction de la plaque (4) ;
- un élément d'engrenage (20), rotatif autour d'un axe de rotation (x), ledit élément d'engrenage (20) entrant en prise avec le secteur denté (18) de sorte qu'une rotation dudit élément d'engrenage (20) provoque une oscillation du secteur denté (18) et de la plaque (4) ; et
- un moteur à engrenage (22), raccordé à l'élément d'engrenage (20) et conçu pour faire tourner ledit élément d'engrenage (20), de sorte que la rotation de l'élément d'engrenage (20) puisse être commandée en vitesse et/ou en position.

2. Dispositif selon la revendication 1, dans lequel ledit élément de contact (10) a un profil de contact (10a), au moins partiellement cylindrique, en contact avec le support fixe (16).

3. Dispositif selon la revendication 2, dans lequel ledit profil de contact cylindrique (10a) est au moins partiellement circulaire et cylindrique.

4. Dispositif selon l'une quelconque des revendications précédentes, dans lequel ledit élément de contact (10) est supporté de manière coulissante sur des rouleaux (24), à leur tour supportés en rotation par le support fixe (16) autour d'axes de rotation respectifs, fixés par rapport audit support fixe (16).

5. Dispositif selon la revendication 4, dans lequel
les rouleaux (24) sont disposés de façon symétrique par rapport à un plan de centre longitudinal (A) du dispositif (9).

6. Dispositif selon l'une quelconque des revendications précédentes, dans lequel une paire d'éléments de contact (10) est fournie, des arches de forme similaire reposant dans au moins un point sur le support fixe (16) et/ou dans au moins deux points sur les rouleaux (24).

7. Dispositif selon l'une quelconque des revendications précédentes, dans lequel le support fixe (16) comprend une paire d'éléments latéraux ayant un profil au moins partiellement complémentaire aux éléments de contact (10), lesdits éléments de contact (10) étant supportés de manière coulissante sur ces éléments latéraux.

8. Dispositif selon l'une quelconque des revendications précédentes, dans lequel le secteur denté (18) est un secteur d'un engrenage annulaire circulaire.

9. Dispositif selon l'une quelconque des revendications précédentes, dans lequel ledit secteur denté (18) est porté par l'élément de contact (10).

10. Dispositif selon la revendication 9, dans lequel ledit secteur denté (18) s'étend à l'intérieur d'une fente en forme d'arc (19), agencée dans l'élément de contact (10), l'élément d'engrenage (20) s'insérant dans ladite fente (19) de manière à s'engrener avec le secteur denté (18).

11. Dispositif selon l'une des revendications 1 à 9, dans lequel ledit secteur denté (18) est embossé sur une paroi latérale de l'élément de contact (10).

12. Dispositif selon l'une quelconque des revendications précédentes, dans lequel l'élément d'engrenage (20) est un pignon.

13. Dispositif selon l'une quelconque des revendications précédentes, comprenant une paire d'éléments de contact (10) avec une surface cylindrique, espacés le long de l'axe de rotation (x).

14. Dispositif selon l'une quelconque des revendications précédentes, dans lequel l'abaissement de la plaque (4), mesuré par la distance du plan de support par rapport au centre de rotation instantané, est compris entre 20 et 80 mm, et est de préférence de 50 mm.

15. Dispositif selon l'une quelconque des revendications précédentes, dans lequel la distance entre la plaque (4) et la surface de support (P) est inférieure à 110 mm.

16. Dispositif selon l'une quelconque des revendications précédentes, dans lequel la plaque (4) peut être rendue oscillante, par rapport à une position de repos, par une mesure comprise entre ± 20° et ± 25°.
